# EUROPÄISCHE PATENTSCHRIFT

(11) **EP 1 982 665 B1**
(45) Veröffentlichungstag und Bekanntmachung des Hinweises auf die Patenterteilung: **13.06.2012**
(21) Anmeldenummer: 08154534.5
(22) Anmeldetag: 15.04.2008
(51) Int. Cl.: A61B 17/86

(54) **Kompressionsschraube mit Drahtelement**
Compression screw with wire element
Vis de compression dotée d'un élément en fil d'acier

(30) Priorität: 20.04.2007 DE 202007005829 U
(43) Veröffentlichungstag der Anmeldung: 22.10.2008
(73) Patentinhaber: Zrinski AG, 78573 Wurmlingen (DE)
(72) Erfinder: Eckhof, Stephan, 78604 Weilheim-Rietheim (DE); Feldhaus, Thomas, 78532 Tuttlingen (DE)

(56) Entgegenhaltungen:
- EP-A- 1 927 322
- FR-A- 2 808 182
- FR-A- 2 840 799
- FR-A- 2 881 942

## Beschreibung

### Technisches Gebiet

Die Erfindung betrifft eine Kompressionsschraube mit einem Drahtelement und einem in Längsrichtung ausgerichtetem Schaft. Der Schaft selbst weist an seinen freien Enden jeweils zwei Bereiche auf, wobei der erste Bereich des Schaftes ein erstes Gewinde und der zweite Bereich ein zweites Gewinde aufweist. Beide Gewinde zeigen unterschiedliche Steigungen.

### Stand der Technik

Kompressionsschrauben dienen dazu, insbesondere Frakturen von Knochen mechanisch zu verbinden. Hierfür ist vorgesehen, dass so genannte Kompressionsschrauben verwendet werden, die derart ausgebildet sind, dass sie einen Schaft aufweisen, wobei an den freien Enden des Schaftes jeweils Bereiche vorgesehen sind, die Gewinde zeigen. Die Gewinde weisen unterschiedliche Steigungen auf, sodass beim Einschrauben der speziellen Kompressionsschraube eine so genannte Kompression eintritt. Unter Kompression ist eine Bewegung zu verstehen, die dann entsteht, wenn durch Einschrauben der Kompressionsschraube in zwei Knochenfragmente diese Knochenfragmente sich aufeinander zu bewegen und somit zueinander komprimiert werden. Die Kompressionsschraube dient daher dazu, die beiden Knochenfragmente unmittelbar zur Anlage zu bringen und zu fixieren.

Aus der FR-A-2 808 182 (NEWDEAL SA [FR]) ist beispielsweise eine Kompressionsschraube mit einem in Längsrichtung ausgerichteten Schaft bekannt, der in zwei Bereiche aufgeteilt ist, und zwar einem ersten und einem zweiten Bereich mit jeweils einem Gewinde. Die beiden Gewinde haben dabei unterschiedliche Steigungen, wobei zur Einführung der Kompressionsschraube in die zu verbindenden Knochenfragmente an ihrem in Einschraubrichtung aufweisenden Ende ein vom Schaft aus durchgehendes starres Drahtelement vorgesehen ist. Dies bedeutet, daß das zur Einführung dienende Drahtelement fester Bestandteil der Kompressionsschraube ist. Eine Sollbruchstelle ist an dem von der Einschraubrichtung wegweisenden Ende der Kompressionsschraube angeordnet.

Ferner besitzt die bekannte Kompressionsschraube für deren Einschraubung an ihrem dem Drahtelement abgewandten Ende eine den Schaft verlängernde stabförmige Einschraubhilfe, die über eine Sollbruchstelle mit dem Schaft verbunden ist. Mit Hilfe eines z. B. elektrischen Schraubwerkzeuges, dessen Spannfutter die stabförmige Einschraubhilfe umgreift, kann dann die Kompressionsschraube verschraubt werden. Danach wird die stabförmige Einschraubhilfe von dem Schaft der Kompressionsschraube an der Sollbruchstelle außerhalb der jeweiligen Wunde abgetrennt.

Weitere derartige Kompressionsschrauben sind auch in den Dokumenten FR-A-2 881 942 (FIXANO SOC PAR ACTIONS SIMPLIF [FR]) und FR-A-2 840 799 (FIXANO [FR]) beschrieben, die ähnlich zu handhaben sind.

Aus der DE 202004020818U U (STRYKER LEIBINGER GMBH ) 26.01.2006 ist eine kanülisierte Kompressionsschraube bekannt. Sie ist ebenfalls als Kompressionsschraube ausgebildet, weist Jedoch die Eigenschaft auf, dass sie einen kanülisierten Hohlraum aufweist, der sich vollständig in Längserstreckung der Kompressionsschraube erstreckt. Zudem sind die freien Enden der Kompressionsschraube jeweils offen, sodass ein Drahtelement durch die kanülisierte Kompressionsschraube hindurch geführt werden kann. Ein solches Drahtelement wird in der Regel bei operativen Eingriffen bei Knochenfrakturen, beispielsweise bei einem Kahnbeinbruch verwendet. Dabei werden zunächst die beiden Knochenfragmente mit dem Drahtelement aufgefädelt und in idealer Passform zusammengefügt beziehungsweise zusammengepuzzelt. Die kanüllsierte Kompressionsschraube wird anschliessend über den Draht "übergeschraubt" und die beiden Knochenfragmente ziehen sich aufgrund der Ausgestaltung der Gewinde zusammen. Die zuvor beschriebene Bewegung tritt ein. Das Drahtelement kann anschliessend entfernt werden.

Es sind auch Operationen bekannt, bei denen auf das Drahtelement verzichtet wird. Das Verzichten auf ein Drahtelement hat jedoch den entscheidenden Nachteil, dass die Führung der Kompressionsschraube innerhalb der Knochenfragmente nicht mehr gewährleistet ist. Durch Einschrauben der Kompressionsschraube besteht die Gefahr, dass diese nicht exakt in die Richtung vorgetrieben wird, die ausgewählt worden ist, beispielsweise hervorgerufen durch unterschiedliche Knochenstrukturen oder ähnliches. Dadurch kann dann nicht die gewünschte Kompression der Knochenfragmente erreicht werden.

Daher wird in der Regel zunächst eine Vorbohrung durchgeführt, in der das Drahtelement entsprechend eingefügt wird und dann mit der speziell ausgebildeten Kompressionsschraube, die auch in der Fachwelt "Herbertschraube" genannt wird, überschraubt.

Bei vielen Operationen findet auch die Anwendung des Drahtelements in der Weise statt, dass dieses auf der einen Seite des Knochenfragmentes eingeführt wird und auf der anderen Seite des Knochenfragmentes wieder hinausgeführt wird. Dadurch kann bei Überschrauben des Drahtelements mit der speziell ausgebildeten kanülisierten Kompressionsschraube das Drahtelement auch in Einschraubrichtung herausgezogen werden. Dies bringt den Vorteil mit sich, dass das der Einschraubrichtung abgewandtes Ende der speziell ausgebildeten kanülisierten Kompressionsschraube als Aufnahmeelement für einen Schraubendreher ausgebildet sein kann.

Die zuvor beschriebenen Verfahren zeigen positive medizinische Erfolge, jedoch die Anwendung des Verfahrens ist sehr komplex und bedarf unterschiedlicher Abhandlungsschritte.

### Aufgabe der Erfindung

Daher ist es Aufgabe der Erfindung, eine Kompressionsschraube bereit zu stellen, mit der eine Kompression von Knochenfragmenten möglich ist und deren Anwendung einfach ausgestaltet ist.

### Lösung der Aufgabe

Die Lösung der Aufgabe besteht darin, dass an einer Kompressionsschraube das als Führungselement dienende Drahtelement über eine Sollbruchstelle einstückig mit dieser verbunden ist.

### Kemgedanke der Lösung

Der Kerngedanke der Lösung ist es, das Verfahren zur Durchführung der Kompression von Knochenfragmenten mittels Vorbohrung, Einführung eines Drahtelements und "Überschrauben" des Drahtelements zu vereinen.

### Vorteile der Erfindung

Der wesentliche Vorteil der Erfindung ist es, dass eine Kompressionsschraube bereitgestellt werden kann, bei der es nicht mehr notwendig ist, diese zu kanülisieren, damit ein entsprechendes Drahtelement aufgenommen werden kann. Trotzdem weist die Kompressionsschraube das Drahtelement als Führungselement auf, sodass wirkungsvoll vermieden werden kann, dass die Kompressionsschraube beim Einschraubvorgang einen anderen Weg einnimmt, als der, der geplant ist. Die Erfindung ist in Patentanspruch 1 beschrieben.

Das Drahtelement ist einstückig mit einer Kompressionsschraube verbunden. Dadurch ist es möglich, Kompressionsschraube und Drahtelement mit einem einzigen Verfahrensgang herzustellen. Vorzugsweise ist vorgesehen, die Kompressionsschraube entsprechend durch Drehen mit einem Werkzeug herzustellen. Alternativ hierzu kann auch vorgesehen sein, die Kompressionsschraube mit einem entsprechenden Spritzgussvorgang herzustellen.

Nach Einführen der Kompressionsschraube in die Knochenfragmente und nach abschliessender Durchführung der Kompression kann durch Festhalten des Drahtelements bei weiterem Verdrehen der Kompressionsschraube das Drahtelement von der Kompressionsschraube entfernt werden. Dies wird dadurch bewirkt, dass zwischen dem Drahtelement und der Kompressionsschraube eine Sollbruchstelle vorhanden ist. Spanbildungen, die beispielsweise bei einem Trennvorgang entstehen, werden dadurch vermieden. Somit dringen keine Fremdkörper ein.

Vorteilhafterweise ist das Drahtelement an seinem freien Ende mit einer Nagelspitze versehen. Dadurch ist es möglich, die Kompressionsschraube zunächst in Form eines Marknagels zu verwenden und in die Knochenfragmente entsprechend einzuschlagen. Sie ist auch dazu geeignet, die Knochenhaut zu durchtrennen.

Vorteilhafterweise ist das der Nagelspitze gegenüberliegende Ende der Kompressionsschraube mit einem Nagelkopf versehen, sodass bei dem Einschlagen die entsprechenden Gewinde nicht beschädigt werden.

Alternativ hierzu kann das freie Ende des Drahtes auch als Lanzenspitze oder Bohrerspitze ausgebildet sein.

Vorzugsweise ist das freie Ende mit einem relativ stumpfen Winkel ausgebildet, so dass sich die Spitze beim Aufsetzen am Knochen gut in diesen einführen lässt. Dadurch wird vermieden, dass die Spitze abrutscht und an der Korfikalis des Knochens entlanggleitet und so etwaige Schäden verursacht.

Eine andere Möglichkeit zum Ansetzen der Kompressionsschraube an dem Knochen ist - neben dem bereits beschriebenen Einschlagen der Kompressionsschraube (bis das Gewinde in Eingriff kommt) - das Eindrehen der Kompressionsschraube bereits von Beginn an. Hierzu ist vorgesehen, die erfindungsgemässe Kompressionsschraube mittels einer speziellen Vorrichtung an einer Bohrmaschine zu adaptieren und durch Betätigen der Bohrmaschine einzudrehen. Vorteilhafterweise kann auf das Bohrsystem zurückgegriffen werden, das bereits für kanülisierte Kompressionsschrauben verwendet wird.

Sobald die entsprechende Platzierung erreicht ist, kann durch Zuhilfenahme eines Eindrehwerkzeuges, beispielsweise eines Schraubendrehers, die Kompressionsschraube weiter eingedreht werden, Die erfindungsgemässe Kompressionsschraube ist derart ausgebildet, dass das Drahtelement die Führungsrichtung vorgibt und die eigentliche Kompressionsschraube diesem Drahtelement "hinterherläuft". Eine Vorbohrung ist zwar möglich, ist jedoch nicht zwingend.

Vorteilhafterweise ist das Drahtelement mit seinem Durchmesser geringer ausgestaltet als der Schaft beziehungsweise das Gewinde. Dies bringt den Vorteil mit sich, dass die Gewinde sich in die Vorbohrung innerhalb der Knochenfragmente, in denen das Drahtelement geführt wird, hineinschneiden und dort eine form- und kraftschlüssige Verbindung herbeiführen können.

Die Längen des Drahtelements können unterschiedlich gestaltet sein. Daher ist es möglich, unterschiedliche Kompressionsschrauben in unterschiedlichen Längen mit unterschiedlich langen Drahtelementen bereitzustellen.

Ein weiterer wesentlicher Vorteil der Erfindung besteht darin, dass auch die Kompressionsschraube zusammen mit dem Drahtelement im minimalinvasiven Bereich verwendet werden kann, da durch das Vorbohren des Drahtelements ausschilesslich zwei Öffnungen, nämlich eine Eintritts- und eine Austrittsöffnung entstehen. Weitere, möglicherweise zu Infektionen führende Öffnungen sind nicht notwendig.

Die mechanische Belastung ist sehr gering. Insbesondere durch Einfügen der Sollbruchstelle zwischen dem Drahtelement und der Kompressionsschraube ist auch ein Abtrennen des Drahtes innerhalb der Vorbohrung in den Knochenfragmenten möglich, sodass nicht die Gefahr bestehen bleibt, dass ein Teil des Drahtes innerhalb des Knochens verbleibt und so möglicherweise auch ein Stück aus dem Knochen ragt und dort entsprechende Schädigungen herbeiführt. Spanbildung wird vermieden.

Ein weiterer wesentlicher Vorteil der Erfindung besteht darin, dass aufgrund des Weglassens einer kanülisierten Ausbildung der Kompressionsschraube diese auch in Ihrem Durchmesser sehr gering ausgebildet werden kann, sodass es auch möglich ist, mit dieser sogenannten "Herbertschraube" Knochenfragmente zu komprimieren, die wesentlich geringere Dicken beziehungsweise Breiten aufweisen.

Weitere vorteilhafte Ausgestaltungen gehen aus der nachfolgenden Beschreibung, den Zeichnungen und den Ansprüchen hervor.

### Kurze Beschreibung der Abbildungen der Zeichnungen

Es zeigen:
- Fig. 1: eine perspektivische Ansicht auf eine erste erfindungsgemässe Kompressionsschraube zusammen mit dem Drahtelement;
- Fig. 2: eine vergrösserte Darstellung des Überganges von der Kompressionsschraube zu dem Drahtelement gemäss Fig. 1;
- Fig. 3: eine schematische Darstellung einer Auswahl von möglichen Ausgestaltungen des freien Endes des Drahtelements.

### Beschreibung mehrerer Ausführungsbeispiele

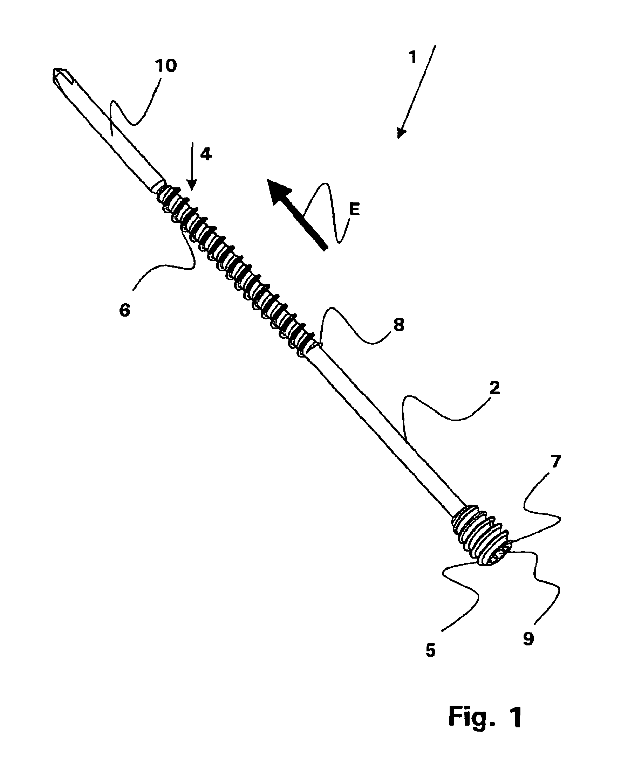

### Allgemein (Fig. 1-3)

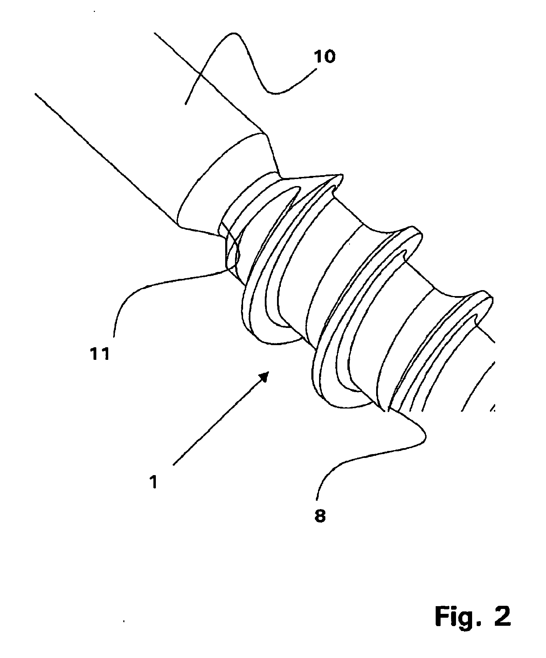

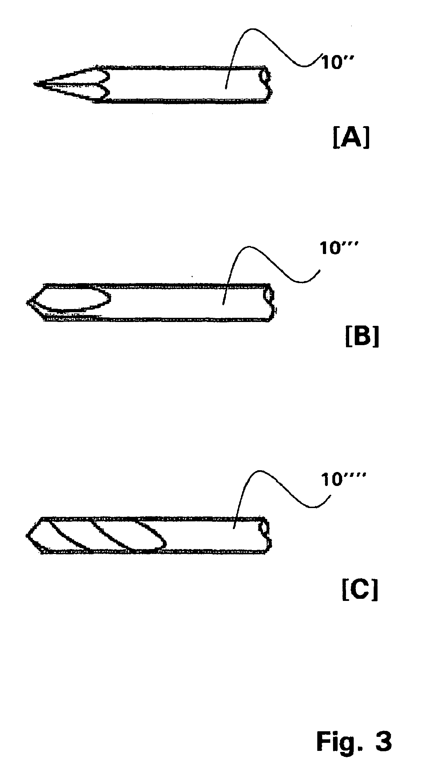

In Fig. 1 und 2 ist die erfindungsgemässe Kompressionsschraube 1 dargestellt. Sie weist einen Schaft 2 auf, wobei an dessen freien Enden 3, 4 jeweils einen ersten Bereich 5 und einen weiteren Bereich 6 mit Gewinden 7, 8 vorgesehen sind. Ferner weist sie ein Drahtelement 10, 10", 10"', 10"" (Fig. 3) auf.

Bei den hier dargestellten Ausführungsbeispielen weisen jeweils der Schaft 2 einen geringeren Durchmesser auf als der Durchmesser der Gewinde 7, 8. Die Gewinde 7, 8 sind derart ausgebildet, dass die gewünschte Kompression eintritt. Dies bedeutet, dass ein erstes und eine zweites Knochenfragment, wobei das erste Knochenfragment über dem ersten Bereich 5 und ein zweites Knochenfragment über den weiteren Bereich 6 der Kompressionsschraube 1 angeordnet ist, beim Eindrehen der Kompressionsschraube 1 sich aufeinander zu bewegen und die Schnittstelle entsprechend komprimiert wird. Der erste Bereich 5, der der Einschraubrichtung E abgewandt ist, weist bei dem hier dargestellten Ausführungsbeispiel einen in Bezug auf seine Länge kürzeren Gewindebereich auf, als der weitere Bereich 6, der der Einschraubrichtung E zugewandt ist.

Zudem ist vorgesehen, dass der der Einschraubrichtung E zugewandte weitere Bereich 6 ein Gewinde 8 aufweist, das in Einschraubrichtung E spitz zu verläuft. Dadurch wird gewährleistet, dass zu Beginn des Eindrehens die Schneidkräfte gering sind und erst mit zunehmendem Eindrehen ansteigen, da zunächst der weitere Bereich 6 und anschliessend, wenn dieser greift und zumindest teilweise eingeschnitten ist, der erste Bereich 5 in Eingriff kommt.

Ferner weist der der Einschraubrichtung E abgewandte Bereich 5 eine Einschraubhilfe 9 auf. Bei dem hier dargestellten Ausführungsbeispiel ist dieses eine Imbuseinrichtung zur Aufnahme eines Imbusschlüssels. Mit Hilfe des Imbusschlüssels kann die Eindrehkraft auf die Kompressionsschraube 1 übertragen werden. Alternativ kann auch eine Spanneinrichtung vorgesehen sein, die als Hilfsmittel zum Spannen in einem Bohrspannfutter dient. Dadurch kann die Kompressionsschraube 1, 1' mit mechanischer Hilfe eingedreht werden.

An der der Einschraubrichtung E zugewandten Seite der Kompressionsschraube 1 ist ferner das erfindungsgemässe Drahtelement 10, 10", 10"', 10"" angeordnet. Es ist bei erfindungsgemäß über eine Sollbruchstelle 11 einstückig mit der Kompressionsschraube 1 verbunden. In Einschraubrichtung E ist das Drahtelement 10, 10", 10"', 10"" in seiner Spitze nagelartig ausgebildet, sodass ein einfacher Vortrieb beim Einschieben des Drahtelements in eine vorzugsweise bereits vorgebohrte Öffnung möglich ist. Alternativ ist auch vorgesehen, keine Vorbohrung durchzuführen. Hierfür ist die Spitze des Drahtelements 10, 10", 10"', 10"" , wie sie exemplarisch in den Fig. 3 [A-C] dargestellt sind, unterschiedlich ausgebildet. In Fig. 3 [A] zeigt ein Drahtelement 10 " mit einer Trokarspitze, Fig. 3 [B] zeigt ein Drahtelement 10"' mit einer Lanzenspitze und Fig. 3 [C] zeigt ein Drahtelement 10"" mit einer Bohrspitze. Diese Ausbildungen sind für alle Ausführungsbeispiele der Kompressionsschraube 1 anwendbar.

### Ausführungsbeispiel (Fig. 1 und 2)

Das Ausführungsbeispiel weist folgende Eigenschaften auf:

Um nach Durchführung der Kompression die Kompressionsschraube 1 ohne das Drahtelement 10 innerhalb der Knochen zu belassen, ist zwischen der Kompressionsschraube 1 und dem Drahtelement 10, so wie es in Fig. 2 exemplarisch dargestellt ist, die Sollbruchstelle 11 vorgesehen. Diese Sollbruchstelle 11 ist derart ausgebildet, dass die Kompressionsschraube 1 bis zum Ansatz des Drahtelements 10 spitz zuläuft und der konstante Querschnitt des Drahtelements 10 unmittelbar an dieser Spitze ansetzt. Dadurch wird ein Sprung des Querschnitts zwischen der Kompressionsschraube 1 und dem Drahtelement 10 herbeigeführt, wobei durch Aufbringen eines entsprechenden Drehmoments ein Abscheren der Kompressionsschraube 1 von dem Drahtelement 10 herbeigeführt wird.

Der Sprung zwischen der Spitze der Kompressionsschraube 1 und dem Durchmesser des Drahtelements 10 ist derart bemessen, dass durch Fixieren des Drahtelementes 10 und Weiterdrehung der Kompressionsschraube 1 ein Drehmoment entsteht, das derart gross ist, dass eine Abscherung erfolgt.

Durch die Abscherung selbst wird vermieden, dass eine Spanentwicklung entsteht.

Das Drahtelement 10 selbst kann nahezu unverändert aus der Vorbohrung der Knochenfragmente entfernt werden.

### Funktionsweise des Ausführungsbeispiels

Die zu komprimierenden Knochenfragmente sind zu lokalisieren und mit einer entsprechenden Durchgangsbohrung zu versehen, wobei sich die Durchgangsbohrung von dem ersten in die weiteren Knochenfragmente erstreckt. Die Durchgangsbohrungen sind derart anzulegen, dass ein Einführen eines Drahtes und ein Ausführen eines Drahtes möglich ist.

Die erfindungsgemässe Kompressionsschraube 1 mit integrativem Bestandteil des Drahtelements 10 wird in die Durchgangsbohrung eingeführt, derart, dass zunächst die Spitze des Drahtelementes 10 die Bohrung lokalisiert und in die Bohrung eingeführt wird. Aufgrund dessen, dass die Durchgangsbohrung geringfügig grösser ist als der Durchmesser des Drahtelements 10, ist ein Einschieben des Drahtelements 10 möglich, bis zu dem Punkt, in dem der weitere Bereich 6 der Kompressionsschraube 1 an die Öffnung der Durchgangsbohrung anstösst. Aufgrund der spitz zulaufenden Ausgestaltung des Gewindes 8 des weiteren Bereiches 6 wird ein zunächst kräftearmer Eindrehvorgang der Kompressionsschraube 1 in die Vorbohrung erreicht. Mit zunehmendem Eindrehen, dass heisst mit zunehmendem Eingreifen von Gewindegängen in den Knochen verstärkt sich die Eindrehkraft, die notwendig ist, um die Kompressionsschraube weiter einzuschrauben. Sobald der weitere Bereich 6 das weitere Knochenfragment erreicht hat, gelangt der Bereich 5 in Eingriff mit dem ersten Knochenfragment und aufgrund der unterschiedlichen Gewindesteigung wird ein Komprimieren der beiden Knochenfragmente erreicht.

Sobald die gewünschte Stellung der Kompressionsschraube 1 erreicht ist, wird das herausstehende Drahtelement 10 fixiert und die Kompressionsschraube 1 um eine geringfügige Drehung weiter gedreht, sodass eine Abscherung an der Sollbruchstelle 11 erfolgt. Das Drahtelement 10 kann nun entsprechend entfernt werden.

Die Wiederherstellung des Knochens ist damit erreicht.

### Bezugszeichenliste

- 1: Kompressionsschraube
- 2: Schaft
- 3: freies Ende des Schafts
- 4: freies Ende des Schafts
- 5: Bereich
- 6: Bereich
- 7: Gewinde
- 8: Gewinde
- 9: Einschraubhilfe
- 10, 10", 10"', 10"": Drahtelement
- 11: Sollbruchstelle
- E: Einschraubrichtung

## Patentansprüche

1. Kompressionsschraube (1) für Knochenfragmente mit in Längserstreckung ausgerichteten Schaft (2), wobei der Schaft (2) in zwei Bereiche aufgeteilt ist und an dem ersten Bereich (5) des Schafts (2) ein erstes Gewinde (7) und im weiteren Bereich (6) ein zweites Gewinde (8) angeordnet ist, wobei die Gewinde unterschiedliche Steigungen aufweisen, So daß beim Eindrehen in Einschraubrichtung eine Kompression der Knochenfragmente erfolgt, und mit einem Drahtelement (10), das an dem in Einschraubrichtung (E) aufweisenden Ende der Kompressionsschraube (1) angeordnet ist, **dadurch gekennzeichnet, dass** das Drahtelement (10, 10", 10"', 10'''') als Führungselement über eine Sollbruchstelle (11) einstückig mit der Kompressionsschraube (1) verbunden ist.

2. Kompressionsschraube nach Anspruch 1, **dadurch gekennzeichnet, dass** die Sollbruchstelle (11) zwischen der Spitze der Kompressionsschraube (1) und dem Drahtelement (10, 10", 10"', 10"") derart bemessen ist, dass diese durch Torsion abscherbar ist.

3. Kompressionsschraube nach Anspruch 2, **dadurch gekennzeichnet, dass** das Drahtelement (10, 10", 10''', 10'''') einen geringeren Durchmesser aufweist als das zweite Gewinde (8) des weiteren Bereiches (6).

4. Kompressionsschraube nach einem der vorhergehenden Ansprüche, **dadurch gekennzeichnet, dass** die Kompressionsschraube (1) an ihrem der Einschraubrichtung (E) abgewandten Ende eine Einschraubhilfe (9) in der Form einer Imbuseinrichtung aufweist.

5. Kompressionsschraube nach einem der vorhergehenden Ansprüche, **dadurch gekennzeichnet, dass** das Drahtelement (10, 10'', 10''', 10'''') unterschiedlich in seiner Länge ausgestaltet ist.

6. Kompressionsschraube nach mindestens einem der vorhergehenden Ansprüche, **dadurch gekennzeichnet, dass** das freie Ende des Drahtelements (10'') nagelartig ausgebildet ist.

7. Kompressionsschraube nach Anspruch 6, **dadurch gekennzeichnet, dass** das Drahtelement (10") eine Nagelspitze aufweist.

8. Kompressionsschraube nach mindestens einem der vorhergehenden Ansprüche, **dadurch gekennzeichnet, dass** das dem Drahtelement (10") gegenüberliegende Ende der Kompressionsschraube (1) ein Nagelkopf umfasst.

## Claims

1. Compression screw (1) for bone fragments, having a shank (2) oriented in the longitudinal direction, wherein the shank (2) is subdivided into two regions and a first thread (7) is arranged in the first region (5) of the shank (2) and a second thread (8) is arranged in the further region (6), wherein the threads have different pitches so that when the screw is screwed in in the screwing-in direction the bone fragments are compressed, and having a wire element (10) which is arranged at that end of the compression screw (1) which is directed in the screwing-in direction (E), **characterized in that** the wire element (10, 10", 10"', 10"") is connected integrally to the compression screw (1) as a guiding element via a predetermined breaking point (11).

2. Compression screw according to Claim 1, **characterized in that** the predetermined breaking point (11) is dimensioned between the tip of the compression screw (1) and the wire element (10, 10", 10"', 10"") such that said compression screw (1) can be sheared off by torsion.

3. Compression screw according to Claim 2, **characterized in that** the wire element (10, 10", 10"', 10"") has a smaller diameter than the second thread (8) of the further region (6).

4. Compression screw according to one of the preceding claims, **characterized in that** the compression screw (1) has a screw-in aid (9) in the form of a hexagon socket device at its end facing away from the screwing-in direction (E).

5. Compression screw according to one of the preceding claims, **characterized in that** the wire element (10, 10", 10"', 10"") is configured with different lengths.

6. Compression screw according to at least one of the preceding claims, **characterized in that** the free end of the wire element (10") is formed in a nail-like manner

7. Compression screw according to Claim 6, **characterized in that** the wire element (10") has a nail tip.

8. Compression screw according to at least one of the preceding claims, **characterized in that that** end of the compression screw (1) opposite the wire element (10") comprises a nail head.

## Revendications

1. Vis de compression (1) pour fragments osseux, comprenant une tige (2) orientée dans la direction longitudinale, la tige (2) étant divisée en deux régions et un premier filetage (7) étant disposé au niveau de la première région (5) de la tige (2) et un deuxième filetage (8) étant disposé au niveau de l'autre région (6), les filetages présentant des pas différents de sorte que lors du vissage dans la direction de vissage, il se produise une compression des fragments osseux, et comprenant un élément en fil métallique (10) qui est disposé à l'extrémité de la vis de compression (1) tournée dans la direction de vissage (E), **caractérisée en ce que** l'élément en fil métallique (10, 10", 10"', 10"") est connecté, en tant qu'élément de guidage, d'une seule pièce à la vis de compression (1) par le biais d'une zone de rupture (11).

2. Vis de compression selon la revendication 1, **caractérisée en ce que** la zone de rupture (11) entre la pointe de la vis de compression (1) et l'élément en fil métallique (10, 10", 10"', 10"") est dimensionnée de telle sorte qu'elle puisse être cisaillée par torsion.

3. Vis de compression selon la revendication 2, **caractérisée en ce que** l'élément en fil métallique (10, 10", 10"', 10"") présente un plus petit diamètre que le deuxième filetage (8) de l'autre région (6).

4. Vis de compression selon l'une quelconque des revendications précédentes, **caractérisée en ce que** la vis de compression (1) présente, à son extrémité opposée à la direction de vissage (E), un auxiliaire de vissage (9) en forme de dispositif pour l'insertion d'une clé.

5. Vis de compression selon l'une quelconque des revendications précédentes, **caractérisée en ce que** l'élément en fil métallique (10, 10", 10"', 10"") est configuré différemment sur sa longueur.

6. Vis de compression selon au moins l'une quelconque des revendications précédentes, **caractérisée en ce que** l'extrémité libre de l'élément en fil métallique (10") est réalisée en forme de clou.

7. Vis de compression selon la revendication 6, **caractérisée en ce que** l'élément en fil métallique (10") présente une pointe de clou.

8. Vis de compression selon au moins l'une quelconque des revendications précédentes, **caractérisée en ce que** l'extrémité de la vis de compression (1) opposée à l'élément en fil métallique (10") comprend une tête de clou.
